))) **Europäisches Patentamt**

(19) **European Patent Office** (11) Numéro de publication: **0 024 963**
**B1**
**Office européen des brevets**

(12) # FASCICULE DE BREVET EUROPEEN

(45) Date de publication du fascicule du brevet:
14.03.84

(51) Int. Cl.³: **A 61 N 1/04,** A 61 M 25/00

(21) Numéro de dépôt: **80401082.5**

(22) Date de dépôt: **22.07.80**

(54) **Dispositif d'introduction et de mise en place d'une sonde ou électrode cardiaque, et sondes ou électrodes utilisées avec ce dispositif.**

(30) Priorité: **26.07.79 FR 7919283**

(43) Date de publication de la demande:
**11.03.81 Bulletin 81/10**

(45) Mention de la délivrance du brevet:
**14.03.84 Bulletin 84/11**

(84) Etats contractants désignés:
**AT BE CH DE GB IT LI LU NL SE**

(56) Documents cités:
**FR - A - 2 187 365**
**FR - A - 2 261 020**
**US - A - 3 754 555**
**US - A - 3 814 104**
**US - A - 3 976 082**

(73) Titulaire: **CARDIOFRANCE - COMPAGNIE FRANCAISE D'ELECTROCARDIOLOGIE, 73, route de Neuilly, F-93160 Noisy Le Grand (FR)**

(72) Inventeur: **Buffet, Jacques, 28, avenue Thiers, F-93340 Le Raincy (FR)**

(74) Mandataire: **Derambure, Christian, Cabinet BUGNION ASSOCIES SARL 116, boulevard Haussmann, F-75008 Paris (FR)**

## Dispositif d'introduction et de mise en place d'une sonde ou électrode cardiaque, et sondes ou électrodes utilisées avec ce dispositif

L'invention concerne un dispositif d'introduction d'une sonde ou électrode ou similaire, souple cardiaque — et de mise en place de la partie extrême libre active de celle-ci dans un corps récepteur notamment le cœur d'un patient recevant des impulsions de stimulation par l'intermédiaire de la sonde ou électrode. L'invention concerne également d'une part des perfectionnements apportés aux sondes, électrodes ou similaires — notamment cardiaques — et d'autre part un outil, faisant partie du dispositif sus-mentionné.

On sait que la mise en service d'un stimulateur cardiaque intra-corporel implique notamment en premier lieu l'introduction d'une sonde ou électrode cardiaque dans une artère ou veine du patient et ensuite la mise en place de la partie extrême libre active de cette sonde ou électrode dans une zone prédéterminée du cœur devant recevoir les impulsions de stimulation, de manière que d'une part cette partie active ne puisse se dissocier de façon intempestive de cette zone du cœur et que d'autre part il y ait entre cette partie active et cette zone un contact électrique satisfaisant. Des problèmes du même type se rencontrennt dans le domaine aénéral de la médecine dans des cas autres que l'implantation d'un stimulateur cardiaque intra-corporel et naturellement la présente invention leur est également applicable.

Jusqu'à présent on utilise généralement des sondes ou électrodes présentant une certaine tenue par exemple du fait qu'elles comportent une armure intérieure ou extérieure. De ce fait, il est possible de donner à la sonde ou électrode une certaine forme ou configuration appropriée par cintrage puis de l'introduire dans l'artère ou la veine appropriée et de la faire ensuite progresser le long de celle-ci en exerçant une poussée axiale sur la seconde partie extrême libre de la sonde ou électrode opposée à la partie active. La mise en place effective de la partie active consiste d'abord à placer celle-ci en regard de la zone choisie du cœur, par manipulation de la seconde partie libre de la sonde ou électrode puis à assurer le contact mécanique et électrique entre cette partie active et la zone choisie du cœur en exerçant une poussée axiale sur la seconde partie libre de la sonde ou électrode.

Un tel procédé implique donc que la sonde ait une certaine tenue de manière qu'une translation axiale effectuée à sa seconde partie libre soit intégralement transmise à la partie active. Or, cette condition ne peut être remplie dans le cas d'une sonde ou électrode constituée essentiellement d'un faisceau de fibres de carbone qui est préférentiellement mise en œuvre pour ses grandes qualités de souplesse donc sa fiabilité, ou conduction électrique ou de compatibilité du corps humain.

La présente invention a donc pour but d'apporter un remède à cette difficulté.

L'invention concerne donc un dispositif d'introduction par voie endocavitaire d'une sonde ou électrode cardiaque souple, constituée d'un faisceau de fibres de carbone et de mise en place de la partie extrême libre active de celle-ci dans le cœur d'un patient.

Ce dispositif est caractérisé en ce qu'il comporte en combinaison en premier lieu une pièce de maintien et d'accrochage associée rigidement à la partie active située à l'extrémité de la sonde et en second lieu un outil d'introduction et de mise en place, amovible, auquel est associée temporairement la sonde ou électrode par sa partie active lors de son introduction et de sa mise en place, comprenant en combinaison des moyens de réception de la pièce, des moyens de manipulation et de guidage associés aux moyens de réception et constitués par un tube souple associé rigidement aux moyens par son extrémité avant, des moyens d'action sous la forme d'un mandrin susceptible de coulisser dans le tube souple, associés aux moyens de réception et propres à permettre de dissocier la pièce des moyens de réception et des moyens de commande à distance des moyens d'action qui empêchent la désolidarisation de la pièce des moyens de réception et d'introduction.

L'invention concerne également les perfectionnements apportés aux sondes ou électrodes. De telles sondes ou électrodes cardiaques sont souples et constituées par un faisceau de fibres de carbone. Elles sont caractérisées par le fait qu'elles constituent en la présence d'une pièce de maintien et d'accrochage associée rigidement à la partie active de la sonde ou électrode et comprenant un corps principal et au moins un organe d'accrochage escamotable.

Les avantages obtenus grâce à l'invention consistent essentiellement en la possibilité d'utiliser des sondes ou électrodes souples, notamment celles constituées d'un faisceau de fibre de carbone en bénéficiant donc de tous les avantages inhérents à de telles sondes ou électrodes. En outre, l'utilisation du dispositif suivant l'invention permet une mise en place beaucoup plus satisfaisante de la partie active de la sonde ou électrode qui est beaucoup mieux associée mécaniquement à la zone déterminée du cœur qu'auparavant, ce qui a également comme conséquence un meilleur contact électrique.

Dans ce qui suit, l'invention est exposée plus en détail à l'aide de dessins représentant seulement un mode d'exécution.

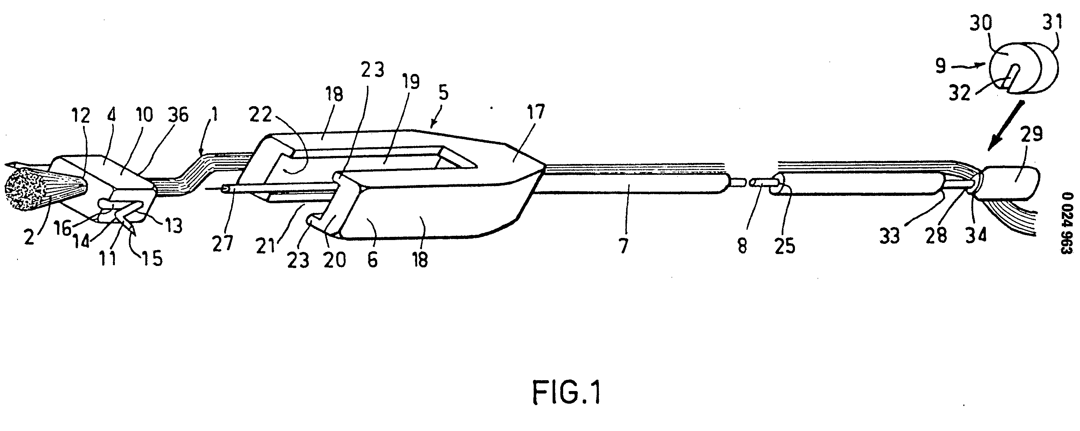

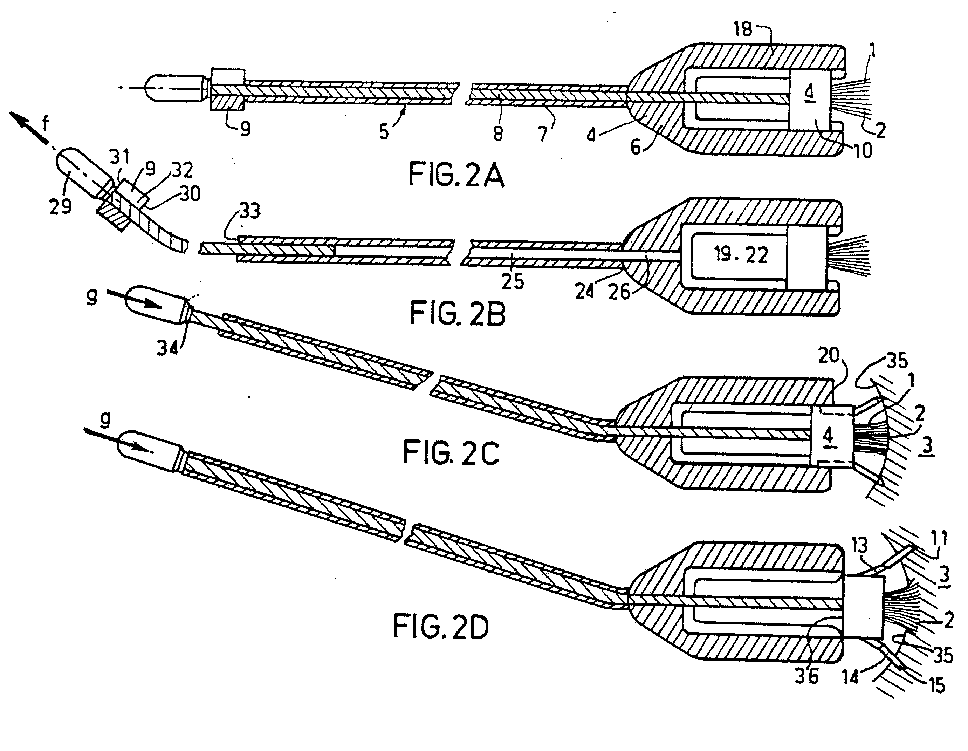

La fig. 1 représente en perspective un dispositif suivant l'invention montrant une sonde ou électrode et un outil; les fig. 2A, 2B, 2C, et 2D sont quatre vues schématiques en coupe par un plan axial illustrant le dispositif suivant l'invention dans quatre phases opératoires.

Les figures représentent un dispositif d'introduction d'une sonde ou électrode 1, souple, notamment cardiaque, et de mise en place de la partie extrême libre active 2 de celle-ci dans un corps récepteur 3, notamment le cœur d'un patient. En particulier, la sonde ou électrode 1 est du type se présentant sous la forme d'un faisceau de fibres de carbone, c'est-à-dire très souple.

Le dispositif suivant l'invention comprend en combinaison en premier lieu une pièce 4 de maintien et d'accrochage, associée rigidement à la partie active 2 de la sonde ou électrode 1 ; et, en second lieu, un outil 5 d'introduction et de mise en place, amovible, auquel est associée temporairement la sonde ou électrode 1 par sa partie active 2, lors de son introduction et sa mise en place. Cet outil 5 comprend en combinaison des moyens 6 de réception de la pièce 4 ; des moyens 7 de manipulation et de guidage des moyens de réception 6, associées à ceux-ci ; des moyens d'action 8 associés aux moyens de réception 6 et propres à permettre de dissocier la pièce 4 et les moyens de réception 6 ; et enfin des moyens 9 de commande à distance des moyens d'action 8.

La pièce 4 est agencée pour une première fonction à savoir d'être maintenue par les moyens de réception 6 et une seconde fonction à savoir de permettre l'accrochage ou l'association ou le maintien sur le corps récepteur 3. La présence de la pièce 4 et ses caractéristiques constituent en soi des perfectionnements apportés aux sondes ou portés aux sondes ou électrodes cardiaques connues.

En ce qui concerne l'outil 5, préférentiellement, les moyens de réception 6 sont associés rigidement aux moyens 7 de manipulation et de guidage. Les moyens 7 visent une première fonction de manipulation — notamment de déplacement en translation — et de guidage des moyens 6. Le cas échéant, les moyens 7 réalisent également une seconde fonction de guidage des moyens d'action 8 qui sont associés aux moyens 7. Quant aux moyens de commande à distance 9, ils sont préférentiellement associés aux moyens d'action 8.

Dans la forme d'exécution particulière préférentielle et non limitative illustrée par les dessins, la pièce 4 comprend un corps principal 10 ayant une forme générale propre à permettre à ce corps d'être réceptionné par les moyens 6 et, associé rigidement au corps 10 au moins un organe d'accrochage 11 élastiquement déformable. Le corps principal 10 a par exemple une forme générale cubique et est réalisé en un matériau non conducteur de l'électricité par exemple en matière plastique. Ce corps 10 est rendu solidaire de la sonde ou électrode 1 proprement dite, notamment du faisceau de fibres de carbone par tout moyen approprié. Par exemple, le faisceau de fibres traverse à force un trou 12 ménagé dans le corps 10 de part en part de celui-ci. La partie extrême libre saillante du faisceau de fibres constitue alors la partie active 2 de la sonde. Préférentiellement, le trou 12 est agencé de manière que le faisceau de fibres à l'arrière du corps 10 ne vienne pas interférer avec les moyens de réception 6. A cet effet et notamment le trou 12 est incurvé à angle droit.

Chaque organe d'accrochage 11 se présente notamment sous la forme générale d'un appendice filiforme comprenant un tronçon de pied 13 associé rigidement au corps 10 et un tronçon d'extrémité 14 terminé par un embout biseauté 15. Cette appendice 11 saille normalement latéralement du corps 10, l'embout 15 étant dirigé vers l'avant et normalement vers l'extérieur de la partie active 2, comme illustré sur la fig. 1. Toutefois, l'appendice 11 est susceptible d'être escamoté et placé en tout ou seulement en partie en ce qui concerne le tronçon de pied 13 dans un logement 16 ménagé dans le corps 10 prenant par exemple la forme d'une rainure. En particulier, l'appendice 11 est escamoté lorsque la pièce 4 est placée dans les moyens de réception 6. L'organe d'accrochage 11 est en tout ou seulement en partie en ce qui concerne les tronçon de pied 13 non conducteur d'électricité par exemple en tout ou partie en matière plastique. Bien entendu, la pièce 4 est agencée de manière à être empêchée de coulisser le long du faisceau de fibres constituant la sonde ou l'électrode 1.

Dans la forme d'exécution particulière illustrée par les dessins, les moyens de réception 6 se présentent sous la forme d'une pièce rigide notamment métallique ayant en élévation une forme générale de U à branches allongées, comprenant une base 17 et deux branches 18 définissant un espace libre central 19 ouvert aux extrémités libres 20 des branches 18 par une ouverture frontale 21. Les branches 18 ont essentiellement comme fonction de permettre de réceptionner la pièce 4, notamment le corps 10. A cet effet, les faces internes 22 des branches 18 sont conformées pour présenter au moins à proximitée des extrémités 20, une forme complémentaire du corps 10. Par exemple, chaque face interne 22, comporte sur son bord longitudinal une saillie 23 empêchant le désengagement intempestif de la pièce 4 une fois associée aux moyens 6. De la sorte, en section droite transversale, les deux branches 18 présentent la forme générale de deux U en regard.

La pièce 4 est destinée à être associée, notamment manuellement aux moyens 6, en faisant pénétrer le corps 10 dans l'espace libre 19 par l'ouverture frontale 21. Lors de cette opération, le ou les organes d'accrochage 11 sont automatiquement escamotés car placés dans le logement 16 et contre la face 22.

Les moyens de manipulation et de guidage 7 se présentent notamment sous la forme d'un tube souple associé rigidement à son extrémité avant 24 à la face arrière de la base 17, par tout moyen approprié. Le tube en question est normalement substantiellement incompressible en sens axial. Pour assurer une grande souplesse du tube, notamment en sens transversal, celui-ci est préférentiellement réalisé substantiellement au moyen d'un fil métallique enroulé à la manière d'un ressort à boudin. Le tube définit donc un conduit central 25 en communication et dans le prolongement d'un conduit 26 axial percé dans les moyens 6 notamment la base 17, et débouchant au fond de l'espace 19 à l'opposé de l'ouverture 21.

Les moyens d'action 8 se présentent notamment sous la forme d'un mandrin susceptible de coopérer et de coulisser dans les conduits 25, 26. Ce mandrin présente en soi une certaine tenue et est donc susceptible de recevoir une certaine déformation permanente résultant notamment d'un cintrage et également de transmettre des efforts exercés axialement à partir de l'une de ses extrémités. Ce mandrin présente une première partie extrême libre active 27 susceptible de faire saillie du conduit 25 en étant logé dans l'espace 19, voire de faire saillie de l'ouverture 21 et une seconde partie extrême libre de manœuvre 28, opposée, à laquelle est associé rigidement, notamment un organe de manœuvre 29 tel qu'une poignée.

Les moyens de commande à distance 9 se présentent notamment sous la forme d'une pièce amovible comprenant deux épaulements opposés 31 et une gorge 32 s'étendant entre les deux épaulements 30, 31, avec laquelle peut coopérer le mandrin des moyens 8. Cette pièce est donc susceptible de venir se placer à cheval sur le mandrin constituant les moyens 8, les épaulements 30, 31 venant respectivement s'appliquer sur la partie extrême libre 33 formant butée du tube 7 opposé à l'extrémité 24 et sur l'organe de manœuvre 29 comportant également un renflement 34 formant butée.

La mise en œuvre du dispositif qui vient d'être décrit est la suivante: initialement on associe la sonde ou électrode 1 en associant la pièce 4 et les moyens 6 comme indqué plus haut, le faisceau de fibres de carbone s'étendant parallèlement à l'extérieur et à l'écart des moyens 6 et 8. Cette association peut être réalisée par tout moyen approprié et notamment manuellement (fig. 2A).

Ensuite, l'opérateur manœouvre la sonde ou électrode 1 essentiellement par l'intermédiaire de l'outil 5 et plus précisément encore par les moyens 7. L'outil 5, plus précisément les moynes 8 sont conformés en fonction de la configuration de la veine ou artère devant être traversée par l'ensemble constitué par la sonde ou électrode 1 et l'outil 5. A cet effet, l'opérateur peut commencer à introduire cet ensemble puis ressortir les moyens 8 des moyens 7 en exerçant une traction axiale dans le sens de la flèche f (fig. 2B). Cette opération permet de couder ou cintrer le mandrin constituant les moyens 8, étant entendu que cette déformation permanente est transmise aux moyens 7. Cette opération de conformation éventuelle des moyens 8 est réalisée en un ou plusieurs temps dans une ou plusieurs zones distinctes de ces moyens (fig. 2B).

Ultérieurement, et par approximations successives, l'opérateur peut amener ainsi la partie active 2 de la sonde 1 en regard d'une zone 35 choisie du coprs récepteur 3. Jusqu'à cette phase, les moyens de commande 9 sont placés de telle manière que les moyens d'action 8 ne provoquent pas la désolidarisation de la pièce 4 et des moyens 6. A cet effet, la pèce constituant les moyens de commande 9 est placée à cheval sur le mandrin des moyens 8, comme indiqué précédemment.

Ultérieurement, l'opérateur peut agir sur les moyens de commande 9 notamment enlever la pièce correspondante ce qui autorise le coulissement du mandrin des moyens 8 dans le sens axial correspondant à la flèche g des fig. 2C et 2D c'est à dire que la partie active 27 du mandrin vient s'appliquer sur le corps 10 notamment sa face postérieure 36, ce qui permet d'amener la partie active 2 au contact de la zone 35, la pièce 4 est d'abord seulement partiellement désengagée, de manière que la partie active 2 soit en contact notamment électrque avec la zone 35 et les organes d'accrochage 11 au moins partiellement escamotés pour s'ancrer provisoirement dans le corps 2, ce qui permet de tester que la zone 35 est correcte (fig. 2C). Ensuite la pièce 4 est totalement libérée et les organes 11 totalement déployés (fig. 2D) et viennent donc s'ancrer efficacement dans la zone 35 en assurant ainsi une tenue mécanique satisfaisante de la sonde. Dans cette phase, la sonde ou électrode 1 est associée au corps 3 dans la zone 35 de façon assurant une bonne tenue mécanique et un bon contact électrique. La sonde ou électrode 1 est dissociée totalement de l'outil 5 qui peut être retiré dans son ensemble en agissant sur les moyens de manipulation 7.

## Revendications

1. Dispositif d'introduction par voie endocavitaire d'une sonde ou électrode cardiaque souple, constituée d'un faisceau de fibres de carbone et de mise en place de la partie extrême libre active (2) de celle-ci dans le cœur (3) d'un patient caractérisé en ce qu'il comporte en combinaison en premier lieu une pièce de maintien et d'accrochage (4) associée rigidement à la partie active (2) située à l'extrémité de la sonde et en second lieu un outil (5) d'introduction et de mise en place, amovible, auquel est associée temporairement la sonde ou électrode (1) par sa partie active (2) lors de son introduction et de sa mise en place, comprenant en combinaison des moyens de réception (6) de la pièce (4), des moyens (7) de manipulation et de guidage associés aux moyens de réception (6) et constitués par un tube souple associé rigidement aux moyens (6) par son extrémité avant, des moyens d'action (8) sous la forme d'un mandrin susceptible de coulisser dans le tube souple associés aux moyens de réception (6) et propres à permettre de dissocier la pièce (4), des moyens de réception (6) et des moyens (9) de commande à distance des moyens d'action (8) qui empêchent la désolidarisation de la pièce (4) des moyens de réception (6) lors de l'introduction.

2. Dispositif selon la revendication 1, caractérisé par le fait que la pièce (4) comprend un corps principal (10) et au moins un organe d'accrochage (11) escamotable.

3. Dispositif selon les revendications 1 et 2, caractérisé par le fait que les moyens de réception (6) se présentent sous la forme d'une pièce ayant une base (17) et deux branches (18) définissant un espace (19) avec lequel peut coopérer le corps (10) de la pièce (4).

4. Dispositif selon les revendications 1 à 3, caractérisé par le fait que le mandrin présente une certaine tenue, ayant une première partie extrême libre active (27) pouvant être logée dans l'espace (19) eet une seconde partie libre de manœuvre (28) à laquelle est associé un organe de manœuvre (29).

5. Dispositif selon les revendications 1 à 4, caractérisé par le fait que les moyens de commande (9) se présentent sous la forme d'une pièce amovible comprenant deux épaulements (30, 31) et une gorge (32) entre ces épaulements.

6. Outil pour l'introductioin endocavitaire et la mise en place d'une sonde ou électrode souple cardiaque, caractérisé par le fait qu'il comporte en combinaison des moyens de réception (6) d'une pièce (4) de maintien et d'accrochage associée à la sonde ou l'électrode (1), des moyens (7) de manipulation et de guidage des moyens de réception (6) associés à ceux-ci et constitués par un tube souple associé rigidement aux moyens (6) par son extrémité avant, des moyens d'action (8) sous la forme d'un mandrin susceptible de coulisser dans le tube souple, associés aux moyens de réception (6) et propres à permettre de dissocier la pièce (4) des moyens de réception (6) et les moyens (9) de commande à distance des moyens d'action (8) qui empêchent la désolidarisation de la pièce (4) des moyens de réception (6) lors de l'introduction.

## Patentansprüche

1. Vorrichtung zum transvenösen Einführen einer biegsamen, aus einem Bündel von Kohlenfasern bestehenden Herz-Sonde oder Elektrode und zum Positionieren des wirksamen freien Endteils (2) davon im Herzen (3) eines Patienten, gekennzeichnet durch die Kombination von, erstens, einem Halterungs- und Verankerungsstück (4), das mit dem am Ende der Sonde sich befindlichen wirksamen Teil (2) starr verbunden ist, und, zweitens, einem abnehmbaren Einführund Positionierwerkzeug (5), mit dem der wirksame Teil (2) der Sonde oder Elektrode (1) während des Einführens und Positionierens vorübergehend verbunden ist, wobei das Einführ- und Positionierwerkzeug (5) die folgende Kombination aufweist: Mitteln (6) zur Aufnahme des genannten Stückes (4); Handhabungs- und Führungsmittel (7), die mit den Aufnahmemittels (6) verbunden sind und die aus einem biegsamen Rohr besteht, dessen vordere Ende mit den genannten Aufnahmemitteln (6) starr verbunden ist; Bestätigungsmittel (8) in Form einer Spindel, die in dem mit den Aufnahmemitteln (6) verbundenen, biegsamen Rohr verschiebbar ist und die dazu angeordnet ist, das Trennen des genannten Stückes (4) von den Aufnahmemitteln (6) zu erlauben; und Mittel (9) zum Fernsteuern der Betätigungsmittel (8) und zu verhindern, daß während des Einführens das genannte Stück (4) von den Aufnahmemitteln (6) getrennt wird.

2. Vorrichtung nach Anspruch 1, dadurch gekennzeichnet, daß das Stück (4) einen Hauptkörper (10) und mindestens ein einfahrbares Verankerungselement (11) enthält.

3. Vorrichtung nach den Ansprüchen 1 und 2, dadurch gekennzeichnet, daß das Aufnahmemittel (6) aus einem Stück besteht, das eine Basis (17) und zwei Arme (18) aufweist, die einen Zwischenraum (19) abgrenzen, mit dem der Körper (10) des Stückes (4) zusammenwirken kann.

4.Vorrichtung nach den Ansprüchen 1 und 3, dadurch gekennzeichnet, daß die Spindel eine gewisse Steifheit aufweist und einen ersten, freien, wirksamen Endteil (27), der sich im Zwischenraum (19) befinden kann, und einen zweiten, freien Bedienungsteil (28), mit dem ein Bedienungsorgan (29) verbunden ist, aufweist.

5. Vorrichtung nach den Ansprüchen 1 bis 4, dadurch gekennzeichnet, daß das Steuerungsmittel (9) die Form eines abnehmbaren Stückes aufweist, das zwei Schultern (30, 31) und dazwischen eine Rinne (32) enthält.

6. Werkzeug zum transvenösen Einführen und Positionieren einer biegsamen Herz-Sonde oder -Elektrode, gekennzeichnet durch die folgende Kombination: Mittel (6) zur Aufnahme eines mit der Sonde oder Elektrode (1) verbundenen Halterungs- und Verankerungsstücks (4); Handhabungs- und Führungsmittel (7) der Aufnahmemittel (6) bestehend aus einem biegsamen Rohr, dessen Vorderende mit den Aufnahmemitteln (6) starr verbunden ist; Bestätigungsmittel (8) in Form einer Spindel, die in dem mit den Aufnahmemitteln (6) verbundenen, biegsamen Rohr verschiebbar ist und die dazu angeordnet ist, das Trennen des genannten Stückes (4) von den Aufnahmemitteln (6) zu erlauben; und Mittel (9) zum Fernsteuern der Betätigungsmittel (8) und zu verbinden, daß während des Einführens das genannte Stück (4) von den Aufnahmemitteln (6) getrennt wird.

## Claims

1. A device for the endocavitary introduction of a flexible cardiac probe or electrode consisting of a bundle of carbon fibres and for positioning the active free end portion (2) thereof in the heart (3) of a patient, characterized in that the device comprises in combination firstly a holding and anchoring member (4) rigidly connected to the active portion (2) situated at the end of the probe, and secondly a detachable introduction and positioning tool (5), to which the active portion (2) of the probe or electrode (1) is temporarily connected during the introduction and positioning thereof, said tool comprising in combination means (6) for receiving said member (4), manipulation and guiding means (7) associated with the receiving means (6) and constituted by a flexible tube having its front end rigidly connected to said receiving means (6), actuating means (8) in the form of a spindle which is slideable in the flexible tube, associated with the receiving means (6) and adapted to permit disconnection of said member (4) from the receiving means (6), and means (9) for remote-controlling the actuating means (8) and which prevents the disconnection of said member (4) from the receiving means (6) during the introduction.

2. A device according to claim 1, characterized in that said member (4) comprises a main body (10) and at least one retractable anchoring element (11).

3. A device according to claims 1 and 2, characterized in that the receiving means (6) are in the form of a piece having a base (17) and two arms (18) defining a space (19) with which the body (10) of said member (4) can cooperate.

4. A device according to claims 1 to 3, characterized in that the spindle has a certain firmness, having an active first free end portion (27) which can be moved into said space (19), and a second free, operating portion (28) to which is connected an operating member (29).

5. A device according to claims 1 to 4, characterized in that the control means (9) is in the form of a detachable member comprising two shoulders (30, 31) and a groove (32) between these shoulders.

6. A tool for the endocavitary introduction and positioning of a flexible cardiac probe or electrode, characterized in that it comprises in combination means (6) for receiving a holding and anchoring member (4) connected to the probe or electrode (1), means (7) for manipulating and guiding the receiving means (6) connected thereto and constituted by a flexible tube having its front end rigidly connected to said receiving means (6), actuating means (8) in the form of a spindle which is slideable in the flexible tube, associated with the receiving means (6) and adapted to permit disconnection of said member (4) from the receiving means (6), and means (9) for remote-controlling the actuating means (8) and which prevents the disconnection of said member (4) from the receiving means (6) during the introduction.

FIG.1

FIG. 2A

FIG. 2B

FIG. 2C

FIG. 2D

0 024 963